# EUROPEAN PATENT SPECIFICATION

(11) **EP 2 582 327 B1**
(45) Date of publication and mention of the grant of the patent: **23.12.2020**
(21) Application number: 11726306.1
(22) Date of filing: 13.06.2011
(51) Int. Cl.: A61F 2/30

(54) **BONE IMPLANT INTERFACE SYSTEM**
VERBINDUNGSSYSTEM FÜR KNOCHENIMPLANTATE
SYSTÈME D'INTERFACE D'IMPLANT OSSEUX

(30) Priority: 18.06.2010 US 818508
(43) Date of publication of application: 24.04.2013
(73) Proprietor: 4-web, Inc., Plano, Texas 75024 (US)
(72) Inventor: HUNT, Jessee, Plano, Texas 75024 (US)
(74) Representative: HGF
(86) International application number: PCT/US2011/040117
(87) International publication number: WO 2011/159587

(56) References cited:
- EP-A1- 0 561 263
- EP-A1- 2 358 309
- WO-A1-2010/080511
- DE-A1-102006 047 663
- DE-C1- 19 543 530
- US-A1- 2010 161 061
- US-B1- 6 206 924

## Description

### BACKGROUND

### 1. Field of the Invention

The present invention relates generally to medical devices and, more particularly to implants.

### 2. Description of Related Art

Implants may be used in human and/or animals to support and/or secure one or more bones. Orthopedic implants are designed to be placed in the body as a replacement for damaged joints or repair of broken bones. For example, a knee replacement procedure may include replacing diseased or damaged joint surfaces of the knee with implants, such as metal and plastic components shaped to allow continued motion of the knee. Although orthopedic implants and procedures are common and have improved over the years, procedures may be susceptible to drawbacks, such as in insufficient interface between the bone and the implant. The bone-implant interface may significantly impact how an implant integrates into the patient's anatomy and, thus, may directly impact long term success of an implant procedure. Providing a sufficient bone-implant interface may be of increased importance where the implant is subject to loading, such as with knee replacements.

The direct structural and functional connection between living bone and the surface of a load-bearing implant is often referred to as osteointegration. Wolfs Law relating to osteointegration is a recognized theory that bone in a healthy person or animal will adapt to the loads it is placed under. If loading on a particular bone increases, the bone will remodel itself over time to become stronger to resist that sort of loading (the external cortical portion of the bone becomes thicker). The converse is true as well: if the loading on a bone decreases, the bone will become weaker due to turnover, it is less metabolically costly to maintain and there is no stimulus for continued remodeling that is required to maintain bone mass.

Current implant designs use various techniques in an attempt to provide strong initial fixation and long-term fixation. For example, joint replacement implants for the knee, hip, shoulder ankle often include posts or screws that provide initial fixation.. Unfortunately, these fixation techniques often exhibit deficiencies, including varied and inadequate stress distribution at the bone-implant interface. Inadequate stress distribution at the bone / implant interface may ultimately lead to a reduction in bone density and thereby cause loosening of the implant In some instances, implants include a porous coating to promote adhesion to the bone Due to multidirectional forces being applied to implants at any given point in time, these coatings may not offer sufficient initial fixation. This lack of fixation may enable micromotion which may lead to irregular bone healing and remodeling, lack of adherence and non-uniformity. Additionally porous coatings may not provide sufficient thickness to facilitate effective bone tissue in-growth within the dynamic environment that implants exist. Such inadequate structural designs often lead to inadequate long term fixation due to issues such as implant component loosening, implant instability, migration of the implant, rotation of the implant, premature wear on articulating surfaces of the bone or implant, periprosthetic fractures of bone at or near the bone-implant interface, as well as other issues.

Accordingly, it is desirable to provide an implant technique that provides a sufficient bone -implant interface.

Each of DE10206047663, EP0561263, DE19543530 and US6206924 discloses an orthopedic implant of the type set forth in the preamble of the accompanying claim 1. EP2358309 forms prior art under Art. 54 (3) EPC and discloses an implant for interfacing with a bone structure including a web structure including a space truss. The space truss includes two or more planar truss units having a plurality of struts joined at nodes and the web structure is configured to interface with human bone tissue.

### SUMMARY

The invention is defined in the appended claims. In addition, in this description various embodiments of implant systems and related apparatus, and methods - not falling under the scope of the claimed invention - of using the same are described. In one embodiment, provided is an orthopedic implant, as set forth in the accompanying claim 1, that includes an implant body having a bone contact surface to be in contact or near contact with a bone structure during use. The implant body comprises a web structure comprising a plurality of planar truss units joined together to form a space truss; a bone interface structure includes a first elongated portion to be at least partially pressed into the bone structure during use, and a second elongated portion to be at least partially pressed into the bone structure during use. The second elongated portion is coupled to the first elongated portion and extends from the first elongated portion at an angle oblique to the first elongated portion. The web structure comprises a plurality of smaller sized space trusses and the bone interface structure comprises a plurality of larger sized space trusses that extend between and above the smaller sized space trusses.

A method that includes providing an orthopedic implant is also disclosed. The implant includes an implant body having a bone contact surface to be in contact or near contact with a bone structure during use, wherein the bone contact surface has a bone interface structure protruding therefrom. The bone interface structure includes a first elongated portion to be at least partially pressed into the bone structure during use, and a second elongated portion to be at least partially pressed into the bone structure during use. The second elongated portion is coupled to the first elongated portion and extends from the first elongated portion at an angle oblique to the first elongated portion. The method also includes inserting the bone interface structure into the bone structure such that that bone contact surface is in contact or near contact with the bone structure.

An implant that includes an implant body having a bone contact surface in contact or near contact with bone structure during use and a bone interface structure protruding from the contact surface, wherein the bone interface structure includes a space truss, and wherein the bone interface structure is disposed within the bone structure during use.

### BRIEF DESCRIPTION OF THE DRAWINGS

Advantages of the present invention will become apparent to those skilled in the art with the benefit of the following detailed description and upon reference to the accompanying drawings in which:
FIG. 1 is a block diagram that illustrates an implant in accordance with one or more embodiments of the present technique;
FIG. 2A is a diagram that illustrates a side view of the implant of FIG. 1A implanted in a bone structure in accordance with one or more embodiments of the present technique;
FIG. 2B is a diagram that illustrate a cross-sectioned view of the implant of FIGS. 1 and 2A taken across line 2B-2B in accordance with one or more embodiments of the present technique;
FIG. 3 is a diagram that illustrates a cut provided in a bone structure in accordance with one or more examples of the present technique;
FIG. 4 is a diagram that illustrates a cutting member in accordance with one or more examples of the present technique;
FIG. 5 is a diagram that illustrates a bone-implant interface including a plurality of bone interface (e.g., rod) structures provided at a contact surface of an implant in accordance with one or more embodiments of the present technique;
FIG. 6 is a diagram that illustrates an implant having bone-implant interface including a multi-layer rod-structure in accordance with one or more embodiments of the present technique;
FIGS. 7A-7G are diagrams that illustrate side views of exemplary two-dimensional rod structures in accordance with one or more embodiments of the present technique;
FIG. 8 is a diagram that illustrates an isometric view of each of the rod structures of FIGS. 7A-7G disposed on a contact surface of a bone-implant interface of an implant in accordance with one or more embodiments of the present technique;
FIGS. 9A-9B are diagrams that illustrate isometric views of a plurality of exemplary three-dimensional rod structures disposed on contact surfaces of bone-implant interfaces of implants in accordance with one or more embodiments of the present technique;
FIGS. 10A and 10B are diagrams that illustrate an isometric view and top view, respectively, of an exemplary implant in accordance with one or more embodiments of the present technique;
FIGS. 11A and 11B are diagrams that illustrate side views of knee implants in accordance with one or more embodiments of the present technique;
FIG. 12 is a diagram that illustrates a side view of an implant in accordance with one or more embodiments of the present technique;
FIG. 13 is a diagram that illustrates a shoulder implant in accordance with one or more embodiments of the present technique; and
FIG. 14 is a flowchart that illustrates an example of a method of implanting an implant in accordance with one or more examples of the present technique.

While the invention is susceptible to various modifications and alternative forms, specific embodiments thereof are shown by way of example in the drawings and will herein be described in detail. The drawings may not be to scale. It should be understood, however, that the drawings and detailed description thereto are not intended to limit the invention to the particular form disclosed, which is defined by the appended claims.

### DETAILED DESCRIPTION OF ILLUSTRATIVE EMBODIMENTS

As discussed in more detail below, the present invention relates to orthopedic implants. In accordance with the invention, an implant includes a bone-implant interface that facilitates integration of the implant with adjacent bone structures. A Done-implant interface provides for effective load transfer between the implant and the adjacent bone. In accordance with the invention, a bone-implant interface includes a surface of the implant having an interface structure (e.g., a rod structure) extending therefrom that is to be disposed in bone structure during use. The rod structure includes a first portion extending away from the surface of the implant and a second portion oriented at least partially oblique to the first portion of the rod structure. In certain embodiments, the rod structure comprises a two dimensional structure extending from the surface. In some embodiments, the rod structure comprises one or more hook shaped members (e.g., V-shaped or U-shaped members) extending from the bone interface surface. In certain embodiments, the rod structure comprises a three dimensional structure extending from the bone interface surface. In some embodiments, the rod structure comprises a plurality of rod members coupled to one another at an apex of the orthopedic implant. In certain embodiments, the rod structure comprises two or more triangular truss structures extending from the bone interface surface, wherein two or more of the triangular truss structures (e.g., triangular planar truss units) share at least one common strut. In certain embodiments, one or more rod members of the rod structure and/or the surface of the implant include a biologic disposed thereon. In some examples, the rod structures are pushed into the bone during implantation. With the rods pushed into the bone the elastic nature of the bone structure may cause the bone to rebound (e.g., grow) in and around the rod structure. This may provide a "grabbing" or "holding" effect of the rod structure which enables the implants initial fixation through integration of the rod structure with adjacent bone structure. Such a grabbing or holding may inhibit movement of the implant. In certain embodiments, the implant may comprises one or more of large joint implants (e.g., a hip and/or knee implant), small joint implants (e.g., shoulder, elbow and/or ankle implants), trauma implants (e.g., shoulder fracture, long bone reconstruction implants and/or intermedullary rod implants), spine implants (e.g., fusion or dynamic implants), cranial maxi facial (e.g., jaw replacement), dental implants.

As used herein the term "truss" refers to a structure having one or more elongate struts connected at joints referred to as nodes. Trusses may include variants of a pratt truss, king post truss, queen post truss, town's lattice truss, planar truss, space truss, and/or a vierendeel truss (other trusses may also be used). Each unit (e.g., region having a perimeter defined by the elongate struts) may be referred to as a "truss unit".

As used herein the term "planar truss" refers to a truss structure where all of the struts and nodes lie substantially within a single two-dimensional plane. A planar truss, for example, may include one or more "truss units" where each of the struts is a substantially straight member such that the entirety of the struts and the nodes of the one or more truss units lie in substantially the same plane. A truss unit where each of the struts is a substantially straight member such that the entirety of the struts and the nodes of the truss units lie in substantially the same plane is referred to as a "planar truss unit".

As used herein the term "space truss" refers a truss having struts and nodes that are not substantially confined in a single two-dimensional plane. A space truss may include two or more planar trusses (e.g., planar truss units) wherein at least one of the two or more planar trusses lies in a plane that is not substantially parallel to a plane of at least one or more of the other two or more planar trusses. A space truss, for example, may include two planar truss units adjacent to one another (e.g., sharing a common strut) wherein each of the planar truss units lie in separate planes that are angled with respect to one another (e.g., not parallel to one another).

As used herein the term "triangular truss" refers to a structure having one or more triangular units that are formed by three straight struts connected at joints referred to as nodes. For example, a triangular truss may include three straight elongate strut members that are coupled to one another at three nodes to from a triangular shaped truss. As used herein a "planar triangular truss" is a triangular truss structure where all of the struts and nodes lie substantially within a single two-dimensional plane. Each triangular unit may be referred to as a "triangular truss unit". A triangular truss unit where each of the struts is a substantially straight member such that the entirety of the struts and the nodes of the triangular truss units lie in substantially the same plane is referred to as a "planar triangular truss unit". As used herein a "triangular space truss" is a space truss including one or more triangular truss units.

As used herein the term "rod" refers to an elongated member. A rod may include cross-sectional shape of varying geometries, such as a circular, oval, triangular, square, rectangular, pentagonal, or the like. A rod may include a longitudinal axis that is straight, substantially straight or curved along its length. As used herein the term "strut" refers to a rod that forms at least a portion of a truss.

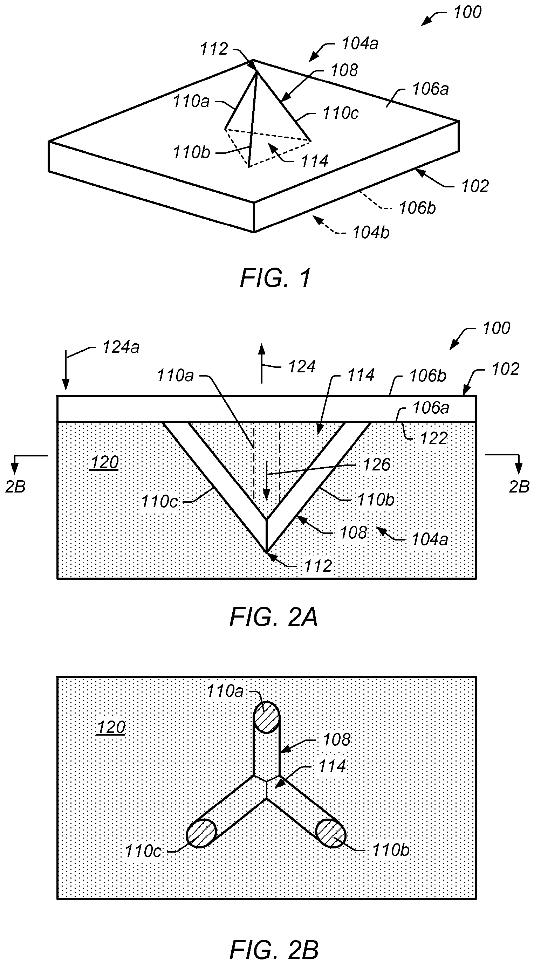

Turning now to the figures, FIG. 1 is a block diagram that illustrates an implant 100 in accordance with one or more embodiments of the present technique. In some embodiments, implant 100 may include a large joint implant (e.g., a hip and/or knee implant), a small joint implant (e.g., shoulder, elbow and/or ankle implants), trauma implants (e.g., shoulder fracture, long bone reconstruction implants and/or intermedullary rod implants), a spine implant (e.g., fusion or dynamic implants), cranial maxi facial implant (e.g., jaw replacement), a dental implant, or the like. In some embodiments, implant 100 may include an intervertebral implant to be implanted between end plates of two adjacent vertebras during a spinal implant procedure. For example, implant 100 may include a fusion implant (e.g., a fusion cage) intended to rigidly fix the relative positions of two adjacent vertebrae, or and dynamic intervertebral device intended to couple to each of the two adjacent vertebrae and to facilitate motion (e.g., flexion, extension, and/or lateral bending) between the two adjacent vertebrae. In some embodiments, implant 100 may include one or more portions of an articulating knee implant. For example, implant 100 may include an upper or lower portion of a knee implant that articulate relative to one another during use, where one or both of the upper and lower portions include bone-implant interfaces that couple implant 100 to bone structures of the knee.

Implant 100 includes one or more bone-interfaces. For example, in the illustrated embodiment, implant 100 includes an implant body 102 having an upper bone-implant interface 104a and a lower bone-implant interface 104b. Implant 100 may include any number of bone-implant interfaces that provide for interface of the implant with bone structure. In some embodiments, upper bone-implant interface 104a may contact and secure to a first adjacent bone structure during use and lower bone- implant interface 104b may contact and secure to a second adjacent bone structure during use. For example, where implant 100 is sandwiched between two adjacent bone structures (e.g., end plates of two adjacent vertebrae), upper bone-implant interface 104a may couple to a portion of the first bone structure disposed above implant 100 and lower bone- implant interface 104b may couple to the second bone structure disposed below implant 100. It will be appreciated that the number and orientation of bone-implant interfaces for a given implant may vary based on the intended applications, and, thus, relative terms such as upper and lower are intended as exemplary and are not intended to be limiting. For example, one or both of the upper and lower bone-implant interfaces 104a and 104b may be oriented such that they are disposed laterally (e.g., as right, left, back and/or front sides of implant body 102). The box-like shape of body 102 is intended to be exemplary and is not intended to be limiting. Body 102 may include any desirable implant construct for the given implant application. For example, spinal implants or knee implants may include a shape, components, and a mechanical construct that provides for motion preservation.

Bone-implant interfaces 104a and 104b include a contact surface. As used herein, the term "contact surface" refers to a portion of an implant intended to be in contact or near contact with an adjacent structure (e.g., a bone structure) and/or to adhere/couple with the adjacent structure when implanted. A contact surface may include an interface plate of an implant, for instance. In the illustrated embodiment, bone-implant interfaces 104a and 104b include an upper contact surface 106a and a lower contact surface 106b, respectively. Contact surfaces 106a and 106b includes portions of implant 100 that are intended to abut and/or integrate with adjacent bone structure when implant 100 is implanted. In some embodiments, implant 100 includes a single contact surface or more than two contact surfaces. Contact surface(s) may take any suitable shape (e.g., a substantially flat planar surface, a curved/contoured surface, ridges, or the like).

In accordance with the invention, bone-implant interfaces includes a structure that facilitates coupling of implant 100 to adjacent bone structure. For example, in the illustrated embodiment, upper bone interface 104a includes contact surface 106a a rod structure 108 extending therefrom. During use rod structure 108 may be pressed into adjacent bone structures. For example, implant 100 may be pressed against a bone structure such that rod structure 108 penetrates into the bone structure and contact face 106a is pressed against a corresponding surface the bone structure. Thus, rod structure 108 may be disposed in the bone structure as discussed in more detail below with respect to FIGS. 2A and 2B.

In some embodiments, some or all of the bone-implant interfaces of an implant include one or more rod structures. For example, in the illustrated embodiment, upper bone-implant interface 104a includes a rod structure 108 disposed thereon. It will be appreciated that although rod structure 108 is illustrated on a single contact surface 106a of a single bone-implant interface 104a, other embodiments may include any number of rod structures disposed at any number of bone-implant interfaces and contact surfaces. For example, in some embodiments, implant 100 includes one or more rod structures disposed on one or both of upper and lower contact surfaces 106a and 106b of bone-implant interfaces 104a and 104b, respectively. Rod structures 108 disposed on both of upper and lower contact surfaces 106a and 106b may be of particular use where implant 100 is intended to span a gap/distance between two adjacent bone structures (e.g., implant 100 is sandwiched between the end plates of two adjacent vertebrae as discussed above).

In accordance with the invention, a rod structure includes at least two rod members (e.g., struts) that extend from a respective contact surface and define region (e.g., an opening or at least a partial opening) that enables bone through growth to facilitate coupling of the rod structure and, thus the implant, to the bone structure. For example, in the illustrated embodiment, rod structure 108 includes a space truss formed of three struts 110a, 110b and 110c. Struts 110a, 110b and 110c may each include substantially straight elongate rod members having a first end coupled to contact surface 106a and a second end coupled to each of the other struts at a vertex 112. Each face of the triangular shaped truss structure includes a planar truss unit having a triangular opening with a perimeter defined by two of struts 110a, 110b and 110c and the adjacent portion of contact face 106a.

As depicted, rod structure 108 includes a generally triangular shaped space truss that defines a four sided, substantially open region (e.g., opening/volume) 114. In some embodiments, opening/volume 114 may facilitate bone growth through rod structure 108, thereby enhancing coupling and integration of implant 100 to the adjacent bone structure. For example, at least a portion of rod structure 108 may be in contact or near contact with the adjacent bone structure, thereby enabling bone growth to extend into and/or through at least a portion of opening/volume 114 of truss structure 108 such that the bone growth interlocks with one or more struts 110a, 110b or 110c of rod structure 108. Interlocking of the bone growth and the struts 110a, 110b or 110c may rigidly fix implant 100 in a fixed location relative to the bone structure.

FIG. 2A illustrates a side view of implant 100 of FIG, 1 implanted in a bone structure 120 in accordance with one or more embodiments of the present technique. FIG. 2B illustrates a cross-sectioned view of implant 100 implanted in a bone structure 120 of FIG. 2A taken across line 2B-2B in accordance with one or more embodiments of the present technique. In the illustrated embodiment, rod structure 108 is disposed into bone structure 120 and contact surface 106a is pressed into contact with face 122 of bone structure 120. Bone structure 120 is disposed in volume 114 of rod structure 108. In some embodiments, bone structure 120 may include bone through growth that grows around struts 110a, 110b and 110c and into opening/volume 114. In some examples, bone structure 120 may include bone growth that encloses slits that are created in bone structure 120 during implanting of rod structure into bone structure 120. As discussed above, bone growth may provide for an interlock of rod structure 108 with bone structure 120 and may, thus, rigidly fix implant 100 in a fixed location relative to the bone structure 120. Rod structure 108 may effectively be 'grabbed' onto by the adjacent bone structure which enables integration of rod structure 108 with the adjacent bone structure 120. In the illustrated embodiment, a force in the direction of arrow 124 acting upon implant 100 may be counteracted by a force in the direction of arrow 126 provided by bone structure 120 resisting movement of implant 100. For example where implant 100 includes a knee implant force 124 may represent an "uplift" force. In some embodiments, a net uplift may be the result of forces acting at a particular portion of implant. For example, uplift may be the result of a downward force on implant 100 as represented by arrow 124a. In response to separating forces, such as those exerted in the direction of arrow 124, bone structure 120 coupled to rod structure 108 and provided in volume 114 may inhibit implant 100 from moving upward in the direction of arrow 124. Similar resistance to lateral/shearing forces (e.g., side to side motion, rotation motion, etc.) may be provided by bone structure 120 coupled to rod structure 108 and provided in opening/volume 114. The load transfer to bone structure 120 in volume 114 through the pulling of strut 110b and 110c in the direction of force 124 may encourage an increase in bone density through remodeling principles found in previously mentioned Wolfs law. In some embodiments, coupling of surface to bone structure 120 (e.g., enhanced via use of a biologic or porous coating) may also provided resistance to motion of implant 100 relative to bone structure 120.

In some embodiments, rod structure 108 extends from contact surface 106a by a distance (e.g., height) that is less than, about the same, the same, or greater than a height/thickness of a body 102 of implant 100. For example, in the illustrated example, rod structure 108 protrudes extends a distance that is about four times the height/thickness of implant body 102. In some embodiments, rod structure 108 may have a height that is about 10%, 15%, 20%, 30%, 40%, 50%, 60%, 70%, 80%, 90%, 100%, 150%, 200%, 250%, 300%, 350%, 400%, 450%, 500%, 550% that of body 102 of implant 100. In some examples, rod structure 108 may have a height that is about 1mm, 2mm, 3mm, 4mm, 5mm, 10mm, 15mm, 20mm, 25mm, 30mm, 40mm, 45mm, 50mm, 55mm, 60mm, 65mm, 70mm, 75mm, 80mm or greater.

In some examples, implant 100 may be pressed into contact with the adjacent bone structure such that at least a portion of rod structure 108 is disposed inside of the adjacent bone structure upon implantation. For example, in some examples, implant 100 may be pressed into contact with bone structure 120 such that vertex 112 pierces into the bone structure and is advanced such that at least a portion of struts 110a, 110b or 110c and opening/volume 114 extend into bone structure 120. Such a technique may encourage bone to grow into and/or through opening/volume 114. In some examples, implant 100 may be advanced/pressed into bone structure 120 until the respective contact surface (e.g., upper contact surface 106a) is in contact or near contact with surface 122 of bone structure 120.

In some embodiments, at least a portion of a bone-implant interface (e.g., the rod structure and/or the contact surfaces) may be coated/treated with a material intend to promote bone growth and/or bone adherence and/or an antimicrobial to prevent infection via the rod structure and/or the contact surface. In some embodiments, at least a portion of a bone- implant interface may be coated with a pain medication (e.g., analgesics) to reduce pain after insertion of the implant into the bone. For example, in some embodiments, at least some or all of the surfaces of struts 110a, 110b or 110c and/or contact surfaces 106a and 106b may be coated with a biologic, a bone growth factor and/or pain medication. In some examples, some or all the bone -implant interface (e.g., the rod structure and/or the contact surfaces) may include a porous surface/coating that facilitates adherence of the contact surface to the adjacent bone structure. For example, some or all of struts 110a, 110b and 110c and/or contact surfaces 106a and 106b may include a porous surface texture to promote bone growth that adheres to rod structure 108 and contact surfaces 106a and 106b.

In some examples, at least a portion of the adjacent bone structure in which a rod structure is to be implanted may be pierced/cut/slit prior to the rod structure being advanced/pressed into the adjacent bone structure. For example, a bone end plate of a vertebra may be cut to accept struts 110a, 110b and 110c of rod structure 108. In some examples, a cutting tool/edge may be used to cut into the adjacent bone structure such that the resulting cuts accommodate portions (e.g., one or more struts or rods) of rod structure 108. For example, where rod structure 108 includes a triangular shape, such as that depicted in FIGS. 1-2 A, one or more complementary cuts may be made into the adjacent bone structure in a complementary Y-shaped pattern.

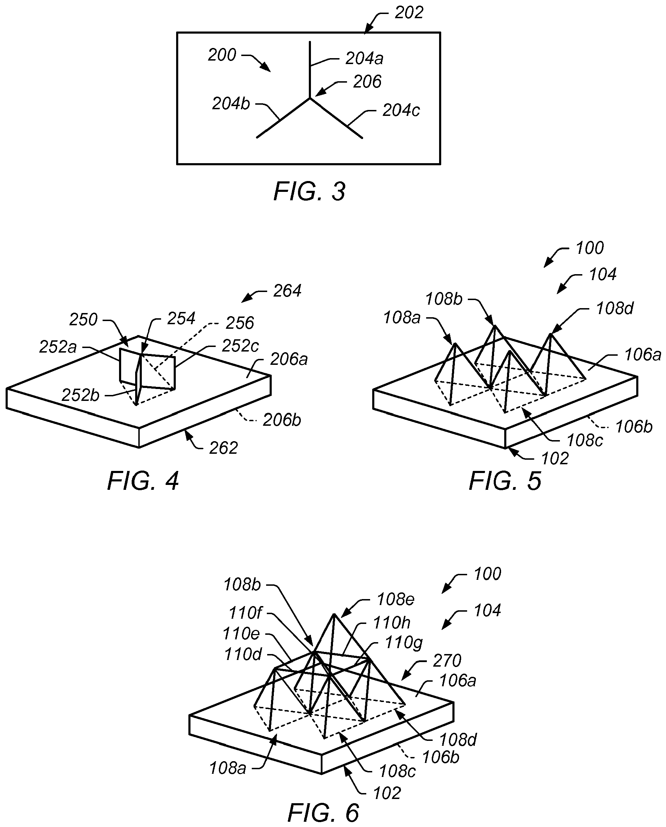

FIG. 3 illustrates a cut 200 that may be provided in a bone structure 202 in accordance with one or more examples of the present technique. Bone structure 202 may be similar to bone structure 120. Cut 220 may be provided prior to or as a result of rod structure 108 being advanced/pressed into the adjacent bone structure 202. FIG. 3 may be representative of an end view of a bone structure. For example, FIG. 3 may be illustrative of the face of a vertebra end plate and a Y-shaped cut extending into the face (e.g., looking upward/downward into the end plate of the vertebrae) that is shaped to accept at least a portion of rod structure 108. In some examples, cut 200 may include one or more segments intended to accommodate one or more portions (e.g., struts or rods) of a rod structure. For example, in the illustrated example, cut 200 includes three slits 204a, 204b and 204c formed in bone structure 202. Slits 204a, 204b and 204c may extend from the face of bone structure 202 into bone structure 202 in a direction substantially perpendicular to a face of bone structure 202 and/or substantially parallel to the intended direction of advancement of struts of rod structure 108 and/or implant 100 into bone structure 202.

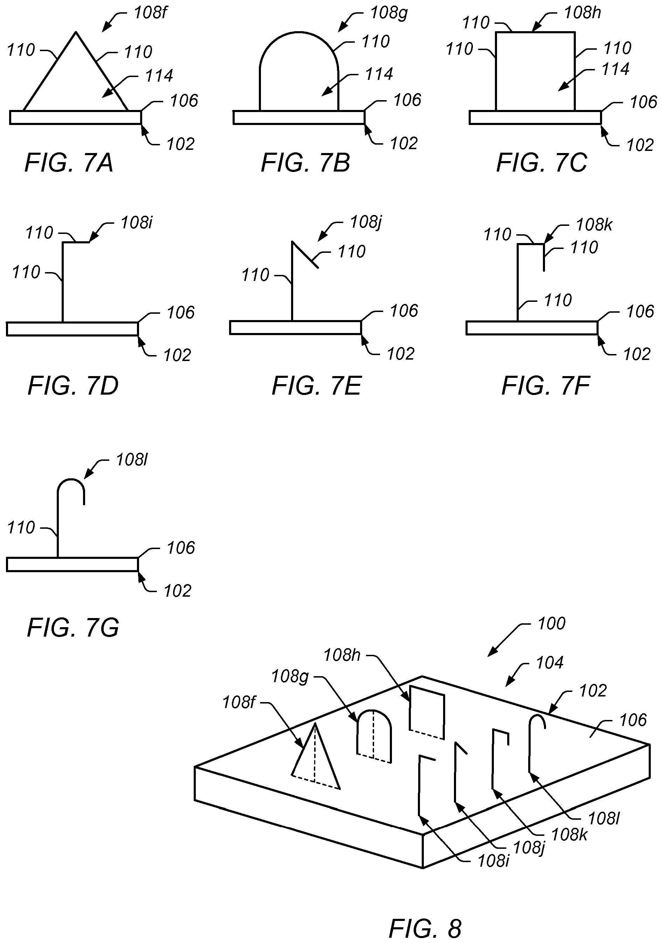

In some examples, slits include cuts into the bone that do not require any bone material to be removed. For example, a sharp cutting edge (e.g., a knife/blade) may be advanced into bone structure 202 to create slits 204a, 204b and 204c, without removing any bone structure 202 or a substantial amount of bone structure 202. During implantation of implant 100 into bone structure 202, struts 110a, 110b or 110c may slide into slits 204a, 204b and 204c, respectively. Cut 200 may be complementary to the shape/orientation of portions (e.g., rods or struts) of rod structure 108. Although the illustrated examples includes three slits oriented at approximately one-hundred twenty degrees relative to one another about a vertex 206, other examples may include any number of slits in any variety of orientation to accommodate one or more struts of a rod structure extending from a contact face of an implant. For example, where rod structure 108 is substantially pyramidal in shape (e.g., see rod structure 108g described below with respect to FIG. 7), cut 200 may include four slits oriented at approximately ninety-degrees relative to one another.

In some examples, cut 200 may be formed by one or more complementary cutting members (e.g., knives/blades) that are pressed, slid, or otherwise advanced into bone structure 202. In some examples, a cutting member includes one or more cutting edges arranged complementary to the profile of the portions (e.g., rods or struts) of rod structure 108 such that advancement of the cutting edge cuts one, a plurality, or all of the slits to accommodate rod structure 108 being advanced/pressed into the bone structure.

FIG. 4 illustrates a cutting member 250 in accordance with one or more examples of the present technique. Cutting member 250 may include three cutting blades 252a, 252b and 252c oriented at approximately one-hundred twenty degrees relative to one another about a vertex 254. In some examples, cutting members 252a, 252b and 252c, are arranged complementary to slits 204a, 204b and 204c of cut 200 and/or struts 110a, 110b or 110c of rod structure 108. Although the illustrated example includes three cutting blades oriented at approximately one-hundred twenty degrees relative to one another about a vertex 254, other examples may include any number of cutting blades in any variety of orientations to accommodate one or more portions (e.g., rods or struts) of rod structure 108 of implant 100. For example, where rod structure 108 is substantially pyramidal in shape (e.g., see rod structure 108b described below with respect to FIG. 7), cutting member 250 may include four cutting blades oriented at approximately ninety-degrees relative to one another.

In some examples, the cutting blades of cutting member 250 may be advanced into bone structure 202 at a depth that is about the same or deeper than a height of rod structure 108. In some examples, the cutting blades may be advanced into bone structure 202 at a depth that is about the same or shallower than a height of rod structure 108. In some examples, a leading edge of the cutting blades may be shaped to be complementary to the shape of the struts. For example, the leading edge of one, a plurality, or all of cutting blades 252a, 252b and 252c, may be angled similar to the angle of struts 110a, 110b or 110c extending from contact surface 106a, as illustrated by dashed line 256 which includes an angle substantially similar to that of a corresponding strut 110c of implant 100.

In some examples, cutting member 250 may be provided as an instrument that is advanced into bone structure 202. In some examples, cutting member 250 may be integrated with or more other devices used during the implantation procedure. For example, during a spinal implant procedure, cutting member 250 may be coupled to a distractor typically positioned between the vertebrae and expanded to set the relative positions of adjacent vertebrae. The force of distraction may act to advance cutting member 250 into bone structure 202. FIG. 4 illustrates cutting member 250 disposed on a top surface 260a of a body 262 of a distractor 264, in accordance with one or more examples of the present technique. In some examples, one or more cutting members may be disposed on other portions of an instruments (e.g., distractor 264), such as a bottom surface 206b. Where distractor 264 includes a distractor (e.g., a spinal distractor), during use, distractor 264 may be disposed between the adjacent bone structures (e.g., adjacent vertebrae) and expanded such that top and bottom surfaces 260a and 260b move away from one another, thereby pressing one or more of cutting members 250 (e.g., on top and/or bottom contact surfaces 260a and 260b) into the adjacent bone structure (e.g., 202) to form one or more cuts (e.g., cut 200) into the bone structure (e.g., end plates of the adjacent vertebrae), where the cuts are intended to accommodate struts (e.g., struts 110a, 110b and 110c) of the rod one or more structures (e.g., rod structure 108) of an implant (e.g., implant 100) to be engaged with the bone structure (e.g., bone structure 120 or 202). In some examples, a distractor may be used to increase a separation distance between two adjacent bone structures (e.g., between end plates of adjacent vertebrae). In some examples, subsequent to making cuts, the distractor is unexpanded and/or removed, and the implant (e.g., 100) is disposed between the bone structures (e.g., in substantially the same position as the distractor) such that one or more rod structures are aligned/engaged with one or more of the resulting cuts. Other examples may include pressing or otherwise advancing cutting member 250 into a bone structure where a rod structure is to be disposed.

Although several of the above embodiments have been described with regard to a single rod structure, other embodiments may include any number and configurations of rod structures. In some embodiments, a plurality of rod structures is provided at one or more bone -implant interfaces of implant 100. FIG. 5 depicts bone -implant interface 104 including a plurality of rod structures 108a, 108b, 108c and 108d provided at upper contact surface 106a of implant 100 in accordance with one or more embodiments of the present technique. In the illustrated embodiment, four rod structures 108a, 108b, 108c and 108d are disposed substantially adjacent one another on upper contact surface 106a of implant 100. Some or all struts of rod structures 108a, 108b, 108c and 108d may share at least one common vertex with another of rod structures 108a, 108b, 108c and 108d at the contact surface 106a. In some embodiments, one, a plurality or all of rod structures are spaced apart from one another. For example, one, a plurality, or all of rod structures 108a, 108b, 108c and 108d may not share a vertex at or near contact surface 106a. In some embodiments, any number of rod structures may be provided on any portion of implant 100. In some embodiments, the shape and orientation of the rod structures may be varied to mimic various desired shapes. For example, in some embodiments, the truss structures of rod structures 108a-108d may be varied in height and/or orientation to provide a curved profile similar to that of a ball and/or a socket of a joint.

In some embodiments, a bone-implant interface includes a plurality of rod structures stacked upon one another to form a web-like truss structure disposed on one or more contact surfaces of implant 100. FIG. 6 illustrates implant 100 having bone-implant interface 104 including a multi-layer rod-structure (e.g., truss/web structure) 270 in accordance with one or more embodiments of the present technique. Multilayer rod structure 270 is disposed at a bone-implant interface of implant 1000. For example, in the illustrated embodiment, multi-layer rod-structure 270 is disposed on contact surface 106a of implant 100. In embodiments, a multi-layer rod-structure includes a plurality of rod structures interconnected and/or stacked upon one another. Stacking of rod structures may address complications in revision procedures where significant bone loss has occurred and there is a need to replace the bone. The first layer of the stacked design may replace the 'height' of the primary bone structure and can be filled with a cement such as PMMA or bone void filler such as calcium phosphate which will remodel into bone over time. The second layer of the stacked structure may provide for fixation and load transferring. For example, in the illustrated embodiment, a triangular rod structure 108e is stacked atop vertices of rod structures 108b, 108c and 108d. In some embodiments, the shape and orientation of the web structure 270 may be varied to mimic various desired shapes. For example, in some embodiments, web structure 270 may be varied in height and/or orientation to provide a curved profile similar to that of a ball and/or a socket of a joint.

In some examples, one or more additional rod members may be provided between one, a plurality, or all of the vertices of rod structures. For example, in the illustrated example, struts 110d- 110h extend between vertices of rod structures 108a-108d. In some embodiments, one or more struts may extend between some or all of the struts at or near the point where they are coupled to the contact face. For example, one or more rod members/struts may extend in place of one or more of the dashed lines illustrated in FIGS. 1, 4 and 5.

Some of the above embodiments have been described with respect to a particular shaped rod structure (e.g., a triangular shaped space truss structure 108) although various shapes of truss structures are contemplated. It will be appreciated that such description is intended to be exemplary and is not intended to be limiting. For example, in some embodiments, rod structure 108 includes a web/truss structure, such as those described in U.S. Provisional Patent Application No. 61/138707 entitled "TRUSS IMPLANT" by Jessee Hunt, filed December 18, 2008 and U.S. Patent Application No. 12/640,825 entitled "TRUSS IMPLANT" by Jessee Hunt, filed December 17, 2009.

In some embodiments, a rod structure includes a two-dimensional rod structure. FIGS. 7A-7G illustrate side views of exemplary two-dimensional rod structures 108f-108l in accordance with one or more embodiments of the present technique. FIG. 8 illustrates an isometric view of each of rod structures 108f-108l of FIGS. 7A-7G disposed on contact surface 106 of bone -implant interface 104 of implant 100 in accordance with one or more embodiments of the present technique. FIG. 7A includes a triangular shaped rod structure 108f that includes two rod members 110 each having ends coupled to one another at a vertex and coupled to contact surface 106 of body 102, defining an opening 114 through which bone growth may occur. Rod structure 108f may include a triangular-shaped planar truss. FIG. 7B includes a U-shaped rod structure 108g that includes a curved rod member 110 having a U-shaped bend at its apex and having ends coupled to contact surface 106 of body 102, defining an opening 114 through which bone growth may occur. In some embodiments, curved rod member 110 may include two or more portions (e.g., rod members) that form the U-shape. For example, a right curved portion may extend from contact surface 106, a left curved portion may extend from contact surface 106 and the two portions may be coupled to one another at an apex of rod structure 108g. Thus, the two curved portions may be oriented relative to one another to form the U-shape defining opening 114. FIG. 7C includes a U- shaped rod structure 108h that includes a plurality of substantially straight rod members 110 having a substantially straight rod member its apex and having two substantially straight rod members at either end coupled to contact surface 106 of body 102, defining an open region 114 through which bone growth may occur. FIG. 7D includes a L-shaped rod structure 108i that includes a first a substantially straight rod member 110 extending from contact surface 106 of body 102 and a second substantially straight rod member 110 oriented at an oblique angle (e.g., substantially perpendicular angle) to the first rod member 100. FIG. 7E includes a hook/barb-shaped rod structure 108j that includes a first a substantially straight rod member 110 extending from contact surface 106 of body 102 and a second substantially straight rod member 110 oriented at an oblique angle (e.g., an acute angle of about forty-five degrees) relative to the first rod member 110. Other embodiments may include various angles of the second rod member relative to the first rod member from about ten degrees to about one hundred seventy degrees (e.g., a second rod member angled oblique about ten, twenty, thirty, forty, fifty, sixty, seventy, eighty, ninety, one hundred, one hundred ten, one hundred twenty, one hundred thirty, one hundred forty, one hundred fifty, one hundred sixty, and/or one seventy degrees relative to the first rod member). FIG. 7F includes a hook-shaped rod structure 108k that includes a first a substantially straight rod member 110 extending from contact surface 106 of body 102, a second substantially straight rod member 110 oriented at an oblique angle (e.g., substantially perpendicular angle) to the first rod member 100, and a third substantially straight rod member 110 oriented substantially parallel to the first rod member 110. Other embodiments may include various angles of the second rod member relative to the first rod member (e.g., a second member angled oblique from about ten degrees to about one-hundred seventy degrees relative to the first rod member - a second member angled oblique about ten, twenty, thirty, forty, fifty, sixty, seventy, eighty, ninety, one hundred, one hundred ten, one hundred twenty, one hundred thirty, one hundred forty, one hundred fifty, one hundred sixty, and/or one seventy degrees relative to the first member) and various angles of the third rod member relative to the second rod member (e.g., a third rod member angled oblique from about ten degrees to about one-hundred seventy degrees relative to the second rod member - a third member angled oblique about ten, twenty, thirty, forty, fifty, sixty, seventy, eighty, ninety, one hundred, one hundred ten, one hundred twenty, one hundred thirty, one hundred forty, one hundred fifty, one hundred sixty, and/or one seventy degrees relative to the second rod member). FIG. 7G includes a hook-shaped rod structure 1081 that includes a rod member having a rounded end curved back towards contact surface 106 of body 102. Accordingly, rod structure 1081 may include rod member 100 having a longitudinal axis that is curved at least at one end to provide a rounded bend that forms a hook-like shape. The bend may include a bend from about ten degrees to about one hundred eighty degrees, as depicted, or more.

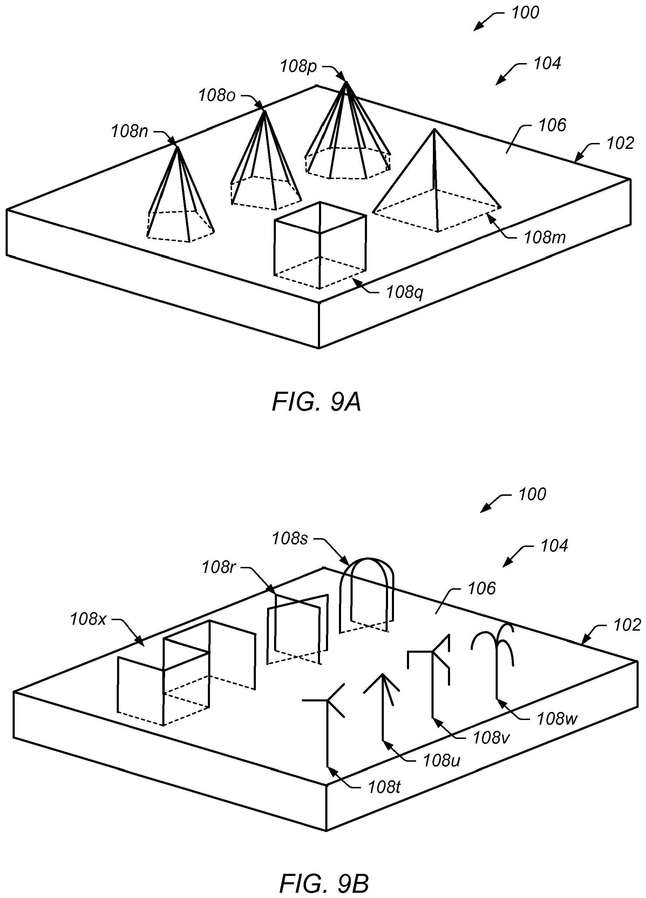

In some embodiments, a rod structure may include a three-dimensional rod structure. For example rod structure may include one or more three-sided triangular shaped space truss structure similar that of rod structure 108 described above with respect to FIGS 1-6. FIGS. 9A-9B illustrate isometric views of a plurality of exemplary three-dimensional rod structures 108m-108w disposed on contact surfaces 106 of bone-implant interfaces 104 of implants 100 in accordance with one or more embodiments of the present technique. Rod structures 108m, 108n, 108n and 108p include a four-sided (e.g., pyramidal) space truss, five-sided space truss, six-sided space truss, and an eight sided space truss, respectively. Rod structure 108q includes a rectangular/square shaped rod structure formed from a plurality of rod members similar to those of rod structure 108h of FIG. 7C. Rod structure 108r includes an X-shaped rod structure formed from a plurality of rod members similar to those of rod structure 108h of FIG. 7C. Rod structure 108s includes an X-shaped rod structure formed from a plurality of curved rod members similar to those of rod structure 108g of FIG. 7B. Rod structure 108t includes a three hook (e.g., treble-hook) shaped rod structure formed from a plurality of rod structures similar to those of rod structure 108i of FIG. 7D. Rod structure 108u includes a treble-hook shaped rod structure formed from a plurality of rod structures similar to those of rod structure 108j of FIG. 7E. Rod structure 108v includes a treble-hook shaped rod structure formed from a plurality of rod structures similar to those of rod structure 108k of FIG. 7F. Rod structure 108w includes a treble-hook shaped rod structure formed from a plurality of rod structures similar to those of rod structure 1081 of FIG. 7G. Rod structure 108x includes an S-shaped rod structure formed from a plurality of rod members similar to those of rod structure 108h of FIG. 7C disposed in a repetitive pattern. Other embodiments may include a random pattern and/or may include a pattern that includes some or all of the other shapes and arrangements of rod structures (e.g., 108-108w) described herein.

In some embodiments, any of the rod structures described herein may be formed via coupling of a plurality of rod members or may be formed of a single rod member that is bent/formed/molded into the provided shape. In some embodiments, rod members (e.g., struts) may have thickness (e.g., diameter) between about 0.25 millimeters (mm) and 5mm (e.g., a diameter of about 0.25mm, 0.5 mm, 0.6 mm, 0.7 mm, 0.8 mm, 0.9 mm, 1 mm, 2 mm, 3 mm, 4 mm, or 5 mm). In some embodiments, a rod structure may have an overall length or width of less than about 1 inch (e.g., a length less than about 0.9 in, 0.8 in, 0.7 in, 0.6 in, 0.5 in, 0.4 in, 0.3 in, 0.2 in, 0.1 in) , with 1 inch being equal to 2.54 cm.

Embodiments may include rod structures having any variety of shapes. For example, other embodiments may include a seven sided space truss and/or space trusses having more than eight sides. In some embodiments, any type, size, number, or combination of number, types and sizes of rod structures may be provided on one, a plurality, or all of the contact faces of an implant as defined in claim 1.

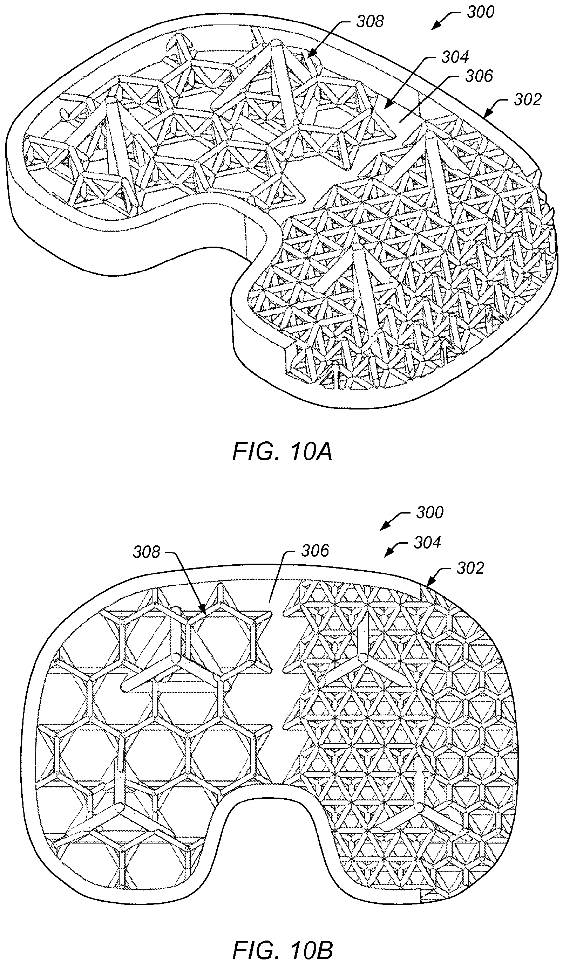

FIGS. 10A and 10B illustrate an isometric view and top view, respectively, of an exemplary implant 300 in accordance with one or more embodiments of the present technique. In some embodiments, implant 300 includes a bone-implant interface 304 that includes a contact face 306 having a plurality of rod structures 308 extending therefrom. In the illustrated embodiment, rod structures 308 include a plurality of different shapes and sizes. For example some of rod structures 308 include smaller sized triangular shaped space trusses, some of rod structures 308 include larger sized triangular shaped space trusses that extend between and above rod members of the smaller sized space trusses, and some of the rod structures 308 include struts arranges in a hexagonal pattern to form six-sided planar trusses of corresponding space trusses. In some embodiments, implant 300 may include a lower portion of a knee implant.

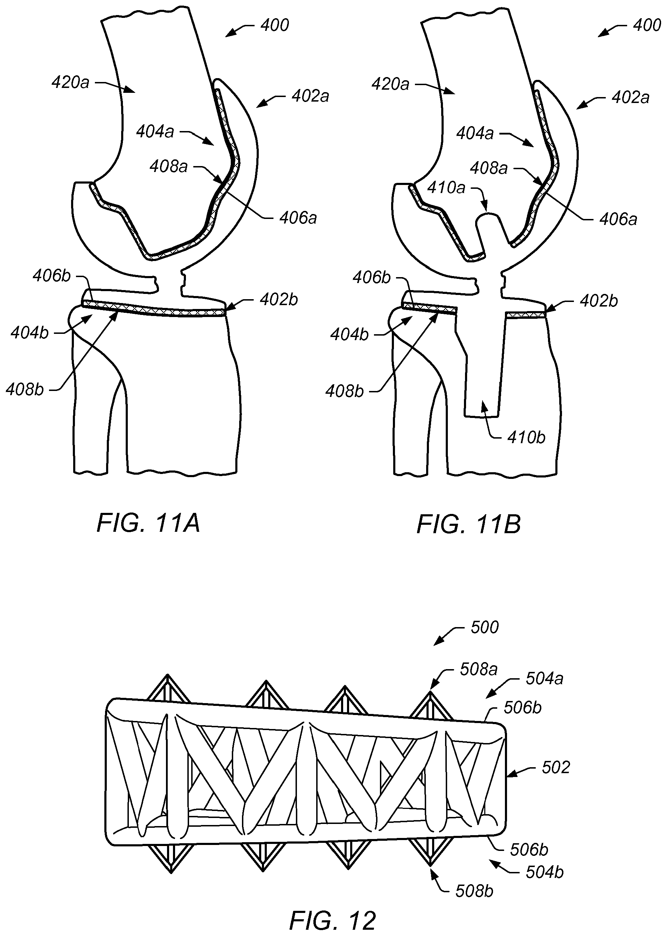

FIG. 11 A illustrates a side view of a knee implant 400 in accordance with one or more embodiments of the present technique. In the illustrated embodiment, implant 400 includes an upper body 402a and a lower body 402b having bone-implant interfaces 404a and 404b respectively. Upper body 402a includes a cup that cradles bone structure 420a. In some embodiments, one or both of bone-implant interfaces 404a and 404b includes a rod structure. For example, in the illustrated embodiment, interfaces 404a and 404b include rod structures 408a and 408b extending from contact surfaces 406a and 406b, respectively.

In some embodiments, rod structures may be used in conjunction with other forms and types of bone -implant interfaces, such as a rod or keel. For example, as depicted in FIG. 11B, upper body 402a may include an elongated rod 410a that is disposed into bone structure 420a and/or lower body 410 may include an elongated rod 410b that is disposed into bone structure 420b. Elongated rod may include a dowel rod, screw, keel or the like.

In some embodiments, implant 100 (e.g., implant body 102) includes a web/truss structure, such as those described in U.S. Provisional Patent Application No. 61/138707 entitled "TRUSS IMPLANT" by Jessee Hunt, filed December 18, 2008 and U.S. Patent Application No. 12/640,825 entitled "TRUSS IMPLANT" by Jessee Hunt, filed December 17, 2009. FIG. 12 illustrates a side view of an implant 500 in accordance with one or more embodiments of the present technique. In the illustrated embodiment, implant 500 includes a body 502 having web/truss structure, and upper and lower bone-implant interfaces 504a and 504b that include a plurality of rod structures 508a and 508b extending from upper and lower contact surfaces 506a and 506b, respectively, of implant 500. Implant 500 may include a spinal implant (e.g., spinal fusion cage, vertebral body replacement (VBR) or spinal motion preservation implant) in some embodiments. For example, upper bone-implant interface 504a may integrate with an endplate of an upper vertebrae (e.g., rod structures 508a may be pressed in the endplate of the upper vertebrae) and lower bone-implant interface 504b may integrate with an endplate of a lower vertebrae (e.g., rod structures 508b may be pressed in the endplate of the vertebrae).

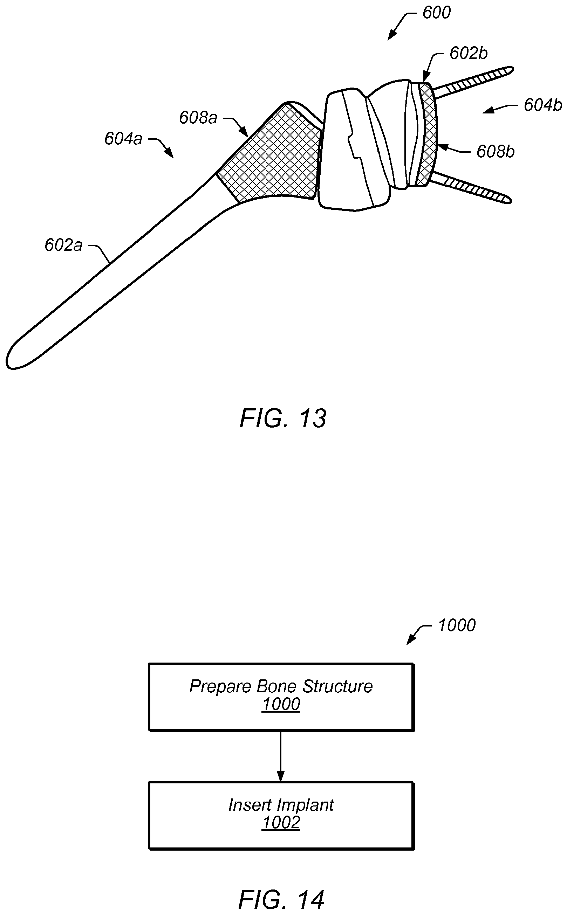

FIG. 13 is a diagram that illustrates a shoulder implant 600 in accordance with one or more embodiments of the present technique. In the illustrated embodiment, implant 600 includes a first body 602a and a second body 602b having bone-implant interfaces 604a and 604b respectively. In some embodiments, one or both of bone-implant interfaces 604a and 604b includes a rod structure. For example, in the illustrated embodiment, interfaces 604a and 604b include rod structures 608a and 608b. In some embodiments, only a rod interface is used to interface with bone. For example, the elongated portion of body 602a may not be present and/or the screws of body 602b may not be present.

FIG. 14 is a flowchart that illustrates an illustrative method 1000 - the method not falling under the scope of the present invention-of implanting an implant in accordance with one or more examples of the present technique. In the illustrated example, method 1000 includes preparing a bone structure, as depicted at block 1002, and inserting an implant (e.g., implant 100), as depicted at block 1002. In some examples, preparing a bone structure includes positioning the bone structure. For example, a distractor (e.g., distractor 262 of FIG. 4) may be used to separate adjacent bone structures such that the implant can be sandwiched between the two adjacent bone structures. In some examples, preparing a bone structure includes cutting/slitting the bone structure to accommodate one or more struts of a rod structure of an implant to be coupled to the bone structure. For example, a cutting member (e.g., cutting member 250) may be advanced into the bone structure to create a cut (e.g., cut 200) including one or more slits (e.g., slits 204a, 204b and 204c). In some examples, distraction and cutting may be provided simultaneously via use of a distractor that includes one or more cutting members coupled to one or more of its contact faces (e.g., distractor 264 having cutting members 250 coupled to both upper and lower faces 206a and 206b).

In some examples, inserting the implant includes positioning the implant (e.g., implant 100) adjacent the bone structure (e.g., bone structure 202), aligning the rod structure (e.g., rod structure 108) with a complementary portion of the bone structure (e.g., cut 200) and/or advancing bone-implant interface (e.g., bone-implant interface 104, 104a or 104b) toward the bone structure such that at least the rod structure is in contact or near contact with the bone structure. In some examples, the implant may be advanced until the contact surface (e.g., contact surfaces 106a and/or 106b) is in contact or near contact with the bone structure, such that at least portion or substantially all of the rod structure is disposed in the bone structure. For example, substantially all of the struts of the truss structure 108 may be disposed in the slits 204a, 204b and 204c provided in the bone structure.

As will be appreciated, method 1000 is exemplary and is not intended to be limiting. One or more of the elements described may be performed concurrently, in a different order than shown, or may be omitted entirely. Method 1000 may include any number of variations. For example, in some examples, rod/struts of rod structure 108 may include a sharp/thin profile such that minimal preparation of the bone structure needed (e.g., cuts do not need to be provided in the bone structure) as the struts of the rod structure may pierce/slice the bone structure as the implant is advanced into contact with the bone surface. Accordingly, in some examples, steps 1002 and 1004 of method 1000 may be combined into a single step.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein. Changes may be made in the elements described herein without departing from the scope of the invention as described in the following claims. Furthermore, note that the word "may" is used throughout this application in a permissive sense (i.e., having the potential to, being able to), not a mandatory sense (i.e., must). The term "include", and derivations thereof, mean "including, but not limited to". As used throughout this application, the singular forms "a", "an" and "the" include plural referents unless the content clearly indicates otherwise. Thus, for example, reference to "a member" includes a combination of two or more members. The term "coupled" means "directly or indirectly connected".

## Claims

1. An orthopedic implant (300), comprising:
an implant body (304) comprising a bone contact surface (306) configured to be in contact or near contact with a bone structure during use, wherein the implant body comprises a web structure (308) comprising a plurality of planar truss units joined together to form a space truss;
a bone interface structure; comprising a first elongated portion configured to be at least partially pressed into the bone structure during use, and a second elongated portion configured to be at least partially pressed into the bone structure during use,
wherein the second elongated portion is coupled to the first elongated portion and extends from the first elongated portion at an angle oblique to the first elongated portion; **characterised in that**
the web structure comprises a plurality of smaller sized space trusses and wherein the bone interface structure comprises a plurality of larger sized space trusses that extend between and above the smaller sized space trusses.

2. The orthopedic implant of claim 1, wherein the bone interface structure comprises a hook-shaped structure extending from the bone contact surface.

3. The orthopedic implant of claim 1, wherein the bone interface structure comprises a three dimensional structure extending from the bone contact surface.

4. The orthopedic implant of claim 3, wherein the bone interface structure comprises two or more triangular truss structures extending from the bone contact surface through the web structure, wherein two or more of the triangular truss structures share at least one common strut.

5. The orthopedic implant of claim 1, wherein one or more portions of the bone interface structure comprise a biologic, growth factor or pain medication disposed thereon.

6. The orthopedic implant of claim 1, wherein the orthopedic implant comprises one or more of a large joint implant, a small joint implant, a trauma implant, a spine implant, an ankle implant, a cranial maxi facial implant and a dental implant.

7. The orthopedic implant of claim 1, wherein the bone interface structure is configured to inhibit uplift of the orthopedic implant during use.

8. The orthopedic implant of claim 1, wherein the bone interface structure is configured to inhibit migration of the orthopedic implant during use.

9. The orthopedic implant of claim 1, wherein the bone interface structure is configured to inhibit rotation of the orthopedic implant during use.

10. The orthopedic implant of claim 1, wherein the bone interface structure has a height that is about 50% to about 150% of a height of the implant body.

11. The orthopedic implant of claim 1, wherein at least one of the first and second elongated portions comprise a longitudinal axis that is curved along its length.

## Patentansprüche

1. Orthopädisches Implantat (300), umfassend:
einen Implantatkörper (304), der eine Knochenkontaktoberfläche (306) umfasst, die so gestaltet ist, dass sie sich im Einsatz in Kontakt oder nahe einem Kontakt mit einer Knochenstruktur befindet, wobei der Implantatkörper eine Bahnstruktur (308) umfasst, die eine Mehrzahl planarer Fachwerkeinheiten umfasst, die miteinander verbunden sind, so dass ein Raumfachwerk gebildet wird;
eine Knochengrenzflächenstruktur; die einen ersten länglichen Teil umfasst, der so gestaltet ist, dass er im Einsatz wenigstens teilweise in die Knochenstruktur gedrückt wird, und einen zweiten länglichen Teil, der so gestaltet ist, dass er im Einsatz wenigstens teilweise in die Knochenstruktur gedrückt wird, wobei der zweite längliche Teil mit dem ersten länglichen Teil gekoppelt ist und sich von dem ersten länglichen Teil in einem schiefen Winkel zu dem ersten länglichen Teil erstreckt; **dadurch gekennzeichnet, dass**
die Bahnstruktur eine Mehrzahl kleinerer Raumfachwerke umfasst, und wobei die Knochengrenzflächenstruktur eine Mehrzahl größerer Raumfachwerke umfasst, die sich zwischen und über den kleineren Raumfachwerken erstrecken.

2. Orthopädisches Implantat nach Anspruch 1, wobei die Knochengrenzflächenstruktur einen hakenförmige Struktur umfasst, die sich von der Knochenkontaktoberfläche erstreckt.

3. Orthopädisches Implantat nach Anspruch 1, wobei die Knochengrenzflächenstruktur eine dreidimensionale Struktur umfasst, die sich von der Knochenkontaktoberfläche erstreckt.

4. Orthopädisches Implantat nach Anspruch 3, wobei die Knochengrenzflächenstruktur zwei oder mehr dreieckige Fachwerkstrukturen umfasst, die sich von der Knochenkontaktoberfläche durch die Bahnstruktur erstrecken, wobei sich zwei oder mehr der dreieckigen Fachwerkstrukturen mindestens eine gemeinsame Strebe teilen.

5. Orthopädisches Implantat nach Anspruch 1, wobei ein oder mehrere Teile der Knochengrenzflächenstruktur einen sich darauf befindenden biologischen Wachstumsfaktor oder ein Schmerzmedikament umfassen.

6. Orthopädisches Implantat nach Anspruch 1, wobei das orthopädische Implantat eines oder mehrere der folgenden umfasst: ein großes Gelenkimplantat, ein kleines Gelenkimplantat, ein Traumaimplantat, ein Wirbelsäulenimplantat, ein Knöchelimplantat, ein craniomaxillofaziales Implantat und/oder ein Zahnimplantat.

7. Orthopädisches Implantat nach Anspruch 1, wobei die Knochengrenzflächenstruktur so gestaltet ist, dass sie im Einsatz eine Hebung des orthopädischen Implantats hemmt.

8. Orthopädisches Implantat nach Anspruch 1, wobei die Knochengrenzflächenstruktur so gestaltet ist, dass sie im Einsatz eine Migration des orthopädischen Implantats hemmt.

9. Orthopädisches Implantat nach Anspruch 1, wobei die Knochengrenzflächenstruktur so gestaltet ist, dass sie im Einsatz eine Rotation des orthopädischen Implantats hemmt.

10. Orthopädisches Implantat nach Anspruch 1, wobei die Knochengrenzflächenstruktur eine Höhe aufweist, die etwa 50% bis etwa 150% einer Höhe des Implantatkörpers entspricht.

11. Orthopädisches Implantat nach Anspruch 1, wobei mindestens einer des ersten und zweiten länglichen Teils eine Längsachse umfasst, die entlang ihrer Länge gekrümmt ist.

## Revendications

1. Implant orthopédique (300), comprenant :
un corps d'implant (304) comprenant une surface de contact avec l'os (306) conçue pour être en contact ou en quasi-contact avec une structure osseuse lors de l'utilisation, le corps d'implant comprenant une structure en toile (308) comprenant une pluralité d'unités en treillis planes jointes ensemble pour former un treillis spatial ;
une structure d'interface osseuse ; comprenant une première partie allongée conçue pour être au moins partiellement pressée dans la structure osseuse lors de l'utilisation, et une seconde partie allongée conçue pour être au moins partiellement pressée dans la structure osseuse lors de l'utilisation, la seconde partie allongée étant accouplée à la première partie allongée et s'étendant depuis la première partie allongée selon un angle oblique par rapport à la première partie allongée ; **caractérisé en ce que**
la structure en toile comprend une pluralité de treillis spatiaux de plus petite taille et
la structure d'interface osseuse comprenant une pluralité de treillis spatiaux de plus grande taille qui s'étendent entre et au-dessus des treillis spatiaux de plus petite taille.

2. Implant orthopédique selon la revendication 1, la structure d'interface osseuse comprenant une structure en forme de crochet s'étendant à partir de la surface de contact avec l'os.

3. Implant orthopédique selon la revendication 1, la structure d'interface osseuse comprenant une structure tridimensionnelle s'étendant à partir de la surface de contact avec l'os.

4. Implant orthopédique selon la revendication 3, la structure d'interface osseuse comprenant au moins deux structures en treillis triangulaires s'étendant de la surface de contact avec l'os à travers la structure en toile, deux des structures en treillis triangulaires ou plus partageant au moins un treillis commun.

5. Implant orthopédique selon la revendication 1, au moins une partie de la structure d'interface osseuse comprenant un agent biologique, un facteur de croissance ou un médicament contre la douleur disposé sur celle-ci.

6. Implant orthopédique selon la revendication 1, l'implant orthopédique comprenant un grand implant articulaire, un petit implant articulaire, un implant de traumatisme, un implant de la colonne vertébrale, un implant de la cheville, un implant crânien maxi facial et/ou un implant dentaire.

7. Implant orthopédique selon la revendication 1, la structure d'interface osseuse étant conçue pour inhiber le soulèvement de l'implant orthopédique pendant l'utilisation.

8. Implant orthopédique selon la revendication 1, la structure d'interface osseuse étant conçue pour inhiber la migration de l'implant orthopédique pendant l'utilisation.

9. Implant orthopédique selon la revendication 1, la structure d'interface osseuse étant conçue pour inhiber la rotation de l'implant orthopédique pendant l'utilisation.

10. Implant orthopédique selon la revendication 1, la structure d'interface osseuse ayant une hauteur qui représente entre environ 50 % et environ 150 % de la hauteur du corps de l'implant.

11. Implant orthopédique selon la revendication 1, au moins une des première et seconde parties allongées comprenant un axe longitudinal qui est courbé sur sa longueur.
